# EUROPEAN PATENT SPECIFICATION

(11) **EP 4 083 073 B1**
(45) Date of publication and mention of the grant of the patent: **09.04.2025**
(21) Application number: 21760732.4
(22) Date of filing: 24.02.2021
(51) Int. Cl.: C12N 5/0783, A61K 39/00, C07K 14/725, C07K 14/715

(54) **NOVEL CHIMERIC ANTIGEN RECEPTOR AND USE THEREOF**
NEUARTIGER CHIMÄRER ANTIGENREZEPTOR UND VERWENDUNG DAVON
NOUVEAU RÉCEPTEUR ANTIGÉNIQUE CHIMÉRIQUE ET UTILISATION ASSOCIÉE

(30) Priority: 28.02.2020 CN 202010128134
(43) Date of publication of application: 02.11.2022
(73) Proprietor: Bioheng Therapeutics Limited, Grand Cayman KY1-1209 (KY)
(72) Inventor: ZHOU, Yali, Nanjing, Jiangsu 210061 (CN); WU, Changshun, Nanjing, Jiangsu 210061 (CN); JIANG, Xiaoyan, Nanjing, Jiangsu 210061 (CN); CHEN, Gong, Nanjing, Jiangsu 210061 (CN)
(74) Representative: Comoglio, Elena
(86) International application number: PCT/CN2021/077580
(87) International publication number: WO 2021/169977

(56) References cited:
- WO-A1-2019/178518
- WO-A1-2019/242339
- WO-A1-2022/166365
- WO-A2-2019/112899
- CN-A- 108 135 988
- CN-A- 109 328 074
- CN-A- 109 880 803
- CN-A- 110 452 882
- CN-A- 110 615 843
- CN-A- 111 269 326

## Description

### Technical Field

The present invention relates to the field of cellular immunotherapy, in particular, to a novel chimeric antigen receptor containing the intracellular region of the cytokine receptor common γ chain (also known as γc chain), and use thereof.

### Background Art

In recent years, cancer immunotherapy technology has been rapidly developed, and particularly immunotherapy associated with chimeric antigen receptor T cell (CAR-T) has achieved excellent clinical effects in the treatment of hematologic tumors. CAR-T cell immunotherapy is to genetically modify T cells *in vitro* to enable the T cells to recognize tumor antigens, and the T cells, after being amplified to a certain amount, are infused back into the patients' body to kill the cancer cells, thus achieving the purpose of treating tumors.

Currently, with the development of technology, four generations of different CAR structures have emerged. The intracellular signaling domain of the firstgeneration CARs contains only a primary signaling domain, for example, CD3 ζ, thus cells carrying the CARs (for example, CAR-T cells) have poor activity, and have short survival time *in vivo.* The second-generation CARs introduce a co-stimulatory domain, for example, CD28 or 4-1BB, so that the cells can continuously proliferate, which enhances the anti-tumor activity. The thirdgeneration CARs contain two co-stimulatory domains (for example, CD28+4-1BB) and the fourth-generation CARs incorporate cytokines or co-stimulatory ligands so as to further enhance T cell response, or incorporate suicide genes so as to make CAR-T cells self-destruct when needed. The second-generation CAR structure is still mostly used in current clinical research.

WO2019178518 discloses a CAR wherein the γc chain is located between CD28 and CD3 ζ.

WO2019242339 discloses a CAR comprising the intracellular domain (ICD) of IL7Ra and the ICD of IL2Rg.

However, CAR-T cell therapy still has some problems in clinical application. for example, massive tumor recurrences in the treatment of hematologic tumors, and a low response rate in solid tumor treatment, which may be caused by complex tumor microenvironment, CAR-T cell depletion, and other factors.

Thus, the existing CAR-T cell therapy still needs to be improved, so as to promote proliferation of CAR-T cells *in vivo,* counteract the immunosuppressive effects of the tumor microenvironment, and further improve the overall therapeutic effect of CAR-T cell therapy against tumors.

### Summary

In a first aspect, the present invention provides a novel chimeric antigen receptor, which contains an antigen-binding region, a transmembrane domain and an intracellular region, the intracellular region consisting of a co-stimulatory domain, an intracellular signaling domain, and an additional signaling region, wherein the additional signaling region consists of the intracellular region of the γc chain, the intracellular signaling domain is the signaling domain of CD3 ζ, the co-stimulatory domain is the co-stimulatory signaling domain of CD137 (4-1BB), characterized in that the co-stimulatory domain, the intracellular signaling domain and the additional signaling region are arranged in order of distance from the cell membrane from nearest to farthest. In a preferred embodiment, the amino acid sequence of the intracellular region is as represented by SEQ ID NO: 16.

In an embodiment, the antigen-binding region is selected from the group consisting of scFv, Fab, single domain antibody, nanobody, antigen binding ligand, recombinant fibronectin domain, anticalin, and DARPIN. Preferably, the antigen-binding region is selected from the group consisting of scFv, Fab, single domain antibody, and nanobody.

In an embodiment, the antigen-binding region is selected from the group consisting of monoclonal antibody, polyclonal antibody, recombinant antibody, human antibody, humanized antibody, murine antibody, and chimeric antibody.

In an embodiment, the antigen-binding region binds to a target selected from the group consisting of: TSHR, CD19, CD123, CD22, BAFF-R, CD30, CD171, CS-1, CLL-1, CD33, EGFRvIII, GD2, GD3, BCMA, GPRC5D, Tn Ag, PSMA, ROR1, FLT3, FAP, TAG72, CD38, CD44v6, CEA, EPCAM, B7H3, KIT, IL-13Ra2, mesothelin, IL-1 1Ra, PSCA, PRSS21, VEGFR2, LewisY, CD24, PDGFR-β, SSEA-4, CD20, Folate receptor α, ERBB2 (Her2/neu), MUC1, EGFR, NCAM, Claudin18.2, Prostase, PAP, ELF2M, Ephrin B2, IGF-I receptor, CAIX, LMP2, gp100, bcr-ab1, tyrosinase, EphA2, Fucosyl GM1, sLe, GM3, TGS5, HMWMAA, o-acetyl-GD2, Folate receptor β, TEM1/CD248, TEM7R, CLDN6, GPRC5D, CXORF61, CD97, CD 179a, ALK, polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, legumain, HPV E6, E7, MAGE A1, ETV6-AML, sperm protein 17, XAGE1, Tie 2, MAD-CT-1, MAD-CT-2, Fos associated antigen 1, p53, p53 mutant, prostate specific protein, survivin and telomerase, PCTA-1/Galectin 8, MelanA/MART1, Ras mutant, hTERT, sarcoma translocation breakpoint, ML-IAP, ERG (TMPRSS2 ETS fusion gene), NA17, PAX3, androgen receptor, Cyclin B1, MYCN, RhoC, TRP-2, CYP1B 1, BORIS, SART3, PAX5, OY-TES 1, LCK, AKAP-4, SSX2, RAGE-1, human telomerase reverse transcriptase, RU1, RU2, intestinal tract carboxylesterase, mut hsp70-2, CD79a, CD79b, CD72, LAIR1, FCAR, LILRA2, CD300LF, CLEC12A, BST2, EMR2, LY75, GPC3, FCRL5, IGLL1, PD1, PDL1, PDL2, TGF β, APRIL, NKG2D, and any combination thereof. Preferably, the target is selected from the group consisting of CD19, CD20, CD22, BAFF-R, CD33, EGFRvIII, BCMA, GPRC5D, PSMA, ROR1, FAP, ERBB2 (Her2/neu), MUC1, EGFR, CAIX, WT1, NY-ESO-1, CD79a, CD79b, GPC3, Claudin18.2, NKG2D, and any combination thereof.

In an embodiment, the transmembrane domain is a transmembrane domain of a protein selected from the group consisting of: TCR α chain, TCR β chain, TCR γ chain, TCR δ chain, CD3 ζ subunit, CD3 ε subunit, CD3 γ subunit, CD3 δ subunit, CD45, CD4, CD5, CD8 α, CD9, CD16, CD22, CD33, CD28, CD37, CD64, CD80, CD86, CD134, CD137, and CD154. Preferably, the transmembrane domain is selected from transmembrane domains of CD8 α, CD4, CD28, and CD278.

In a second aspect, the present invention further provides a nucleic acid containing a sequence encoding the chimeric antigen receptor of the present invention, a vector containing the nucleic acid, and an immune cell containing the nucleic acid or vector.

In an embodiment, the present invention provides a nucleic acid, which contains a sequence encoding the chimeric antigen receptor of the present invention. Preferably, the nucleic acid is DNA or RNA, more preferably mRNA.

In an embodiment, the present invention provides a vector containing the above nucleic acid. Specifically, the vector is selected from the group consisting of linear nucleic acid molecule, plasmid, retrovirus, lentivirus, adenovirus, vaccinia virus, Rous Sarcoma Virus (RSV), polyoma virus and adeno-associated virus (AAV), bacteriophage, phagemid, cosmid or artificial chromosome. In some embodiments, the vector further contains elements such as an origin of autonomous replication in an immune cell, a selectable marker, a restriction enzyme cleavage site, a promoter, a poly-A tail (polyA), 3' UTR, 5' UTR, an enhancer, a terminator, an insulator, an operon, a selectable marker, a reporter gene, a targeting sequence, and/or a protein purification tag. In a specific embodiment, the vector is an *in vitro* transcription vector.

In an embodiment, the present invention provides an immune cell containing the nucleic acid or vector of the present invention, which can express the chimeric antigen receptor of the present invention. In a specific embodiment, the immune cell is selected from the group consisting of a T cell, a macrophage, a dendritic cell, a monocyte, an NK cell, or an NKT cell. Preferably, the T cell is a CD4+/CD8+ double positive T cell, a CD4+ helper T cell, a CD8+ T cell, a tumor infiltrating cell, a memory T cell, a naive T cell, a γδ-T cell, or an αβ-T cell.

In a third aspect, the present invention provides a pharmaceutical composition, containing the chimeric antigen receptor or the nucleic acid encoding the same, the vector or the immune cell containing them of the present invention as defined above, and one or more pharmaceutically acceptable excipients.

In a fourth aspect, the present invention provides the pharmaceutical composition of the invention as described above for use in treating a subject with cancer, including administering to the subject an effective amount of the chimeric antigen receptor, the immune cell or the pharmaceutical composition according to the present invention.

In an embodiment, the cancer is selected from the group consisting of: blastoma, sarcoma, leukemia, basal cell carcinoma, biliary tract cancer, bladder cancer, bone cancer, brain and CNS cancer, breast cancer, peritoneal cancer, cervical cancer, choriocarcinoma, colon and rectal cancer, connective tissue cancer, cancer of digestive system, endometrial cancer, esophageal cancer, eye cancer, head and neck cancer, stomach cancer, glioblastoma (GBM), liver cancer, hepatoma, intraepithelial tumor, kidney cancer, larynx cancer, leukemia, liver tumor, lung cancer, lymphoma, melanoma, myeloma, neuroblastoma, oral cancer, ovarian cancer, pancreatic cancer, prostate cancer, retinoblastoma, rhabdomyosarcoma, rectal cancer, cancer of respiratory system, salivary gland cancer, skin cancer, squamous cell carcinoma, stomach cancer, testicular cancer, thyroid cancer, uterine or endometrial cancer, malignant tumor of urinary system, vulval cancer and other cancer and sarcoma, and B cell lymphoma, mantle cell lymphoma, AIDS-related lymphoma, and Waldenstrom macroglobulinemia, chronic lymphocytic leukemia (CLL), acute lymphocytic leukemia (ALL), B cell acute lymphocytic leukemia (B-ALL), T cell acute lymphocytic leukemia (T-ALL), B cell prolymphocytic leukemia, blast cell plasmacytoid dendritic cell tumor, Burkitt lymphoma, diffuse large B cell lymphoma, follicular lymphoma, chronic myelogenous leukemia (CML), malignant lymphoproliferative disorder, MALT lymphoma, hairy cell leukemia, marginal zone lymphoma, multiple myeloma, myelodysplasia, plasmablastic lymphoma, preleukemia, plasmacytoid dendritic cell tumor, and post-transplant lymphoproliferative disorder (PTLD). Preferably, the disease which can be treated with the chimeric antigen receptor, the nucleic acid, the vector, the immune cell, or the pharmaceutical composition of the present invention is selected from the group consisting of: leukemia, lymphoma, multiple myeloma, brain glioma, pancreatic cancer, stomach cancer, and so on.

### Detailed Description

Unless otherwise indicated, all scientific and technical terms used herein have the same meaning as commonly understood by those of ordinary skill in the art to which the present invention belongs.

### Chimeric antigen receptor

As used herein, the term "chimeric antigen receptor" or "CAR" refers to an artificially constructed hybrid polypeptide, where a basic structure of the hybrid polypeptide includes an antigen-binding region (e.g., an antigen binding portion of an antibody), a transmembrane domain, a co-stimulatory domain, and an intracellular signaling domain. CAR can redirect the specificity and reactivity of T cell and other immune cells to a selected target in a non-MHC-restricted manner by utilizing the antigen binding properties of monoclonal antibodies. Non-MHC-restricted antigen recognition gives CAR-expressing T cell the ability to recognize an antigen independent of antigen treatment, thus bypassing the major mechanism of tumor escape. Besides, when expressed within T cell, CAR advantageously does not dimerize with α chain and β chain of the endogenous T cell receptor (TCR). In addition to the basic structures such as the antigen-binding region, the transmembrane domain, the co-stimulatory domain, and the intracellular signaling domain, the novel chimeric antigen receptor of the present invention further includes an additional signaling region consisting of the intracellular region of the γc chain.

As used herein, "antigen-binding region" refers to any structure or a functional variant thereof that can bind to an antigen. The antigen-binding region may be an antibody structure, including, but not limited to, monoclonal antibody, polyclonal antibody, recombinant antibody, human antibody, humanized antibody, chimeric antibody, and functional fragment thereof. For example, the antigen-binding region includes, but is not limited to, Fab, single chain antibody fragment (scFv), single domain antibody (sdAb), nanobody (Nb), antigen binding ligand, recombinant fibronectin domain, anticalin, DARPIN, and so on, preferably selected from Fab, scFv, sdAb, and nanobody. In the present invention, the antigen-binding region may be monovalent or bivalent, and may be a monospecific, bispecific or multispecific antibody. In another embodiment, the antigen-binding region also may be a specific binding polypeptide or receptor of a specific protein, wherein the specific protein is, for example, PD1, PDL1, PDL2, TGF β, APRIL, and NKG2D.

"Fab" refers to any one of two identical fragments produced after an immunoglobulin molecule is cleaved by papain, and consists of an intact light chain and a heavy chain N-terminal part linked by a disulfide bond, wherein the heavy chain N-terminal part includes a heavy chain variable domain and CH1. Compared with intact IgG, Fab lacks an Fc fragment, has relatively high fluidity and tissue penetration ability, and can univalently bind to an antigen without mediating antibody effects.

"Single chain antibody" or "scFv" is an antibody composed of antibody heavy chain variable domain (VH) and light chain variable domain (VL) linked by a linker. The optimal length and/or amino acid composition of the linker can be selected. The length of the linker will significantly affect the variable domain folding and interaction of the scFv. In fact, intrachain folding can be prevented if a shorter linker (e.g. 5-10 amino acids) is used. Regarding the selection of size and composition of the linker, see, e.g., Hollinger et al., 1993 Proc Natl Acad. Sci. U.S.A. 90:6444-6448; US patent applications with publication Nos. 2005/0100543, 2005/0175606, 2007/0014794; and PCT applications with publication Nos. WO2006/020258 and WO2007/024715.

"Single domain antibody" or "sdAb" refers to an antibody that naturally lacks light chain, and the antibody contains only one heavy chain variable domain (VHH) and two conventional CH2 and CH3 regions, also known as "heavy chain antibody".

"Nanobody" or "Nb" refers to a VHH structure that is individually cloned and expressed, which has structural stability and binding activity to an antigen comparable to those of the original heavy chain antibody, and is the smallest unit currently known to be capable of binding to a target antigen.

The term "functional variant" or "functional fragment" refers to a variant that substantially contains the amino acid sequence of a parent, but, compared with the parent amino acid sequence, contains at least one amino acid modification (i.e., substitution, deletion, or insertion), provided that the variant retains the biological activity of the parent amino acid sequence. In an embodiment, the amino acid modification is preferably a conservative modification.

As used herein, the term "conservative modification" refers to amino acid modification that does not significantly affect or alter the binding characteristics of the antibody or antibody fragment containing the amino acid sequence. These conservative modifications include amino acid substitution, addition, and deletion. The modifications can be introduced into the chimeric antigen receptor of the present invention by standard techniques known in the art, such as sitedirected mutagenesis and PCR-mediated mutagenesis. The conservative amino scid substitution is a substitution in which the amino acid residue is replaced with an amino acid residue having a similar side chain. Amino acid residue families having a similar side chain have been defined in the art, including basic side chain (e.g., lysine, arginine, histidine), acidic side chain (e.g., aspartic acid, glutamic acid), uncharged polar side chain (e.g., glycine, asparagine, glutamine, serine, threonine, tyrosine, cysteine), nonpolar side chain (e.g., alanine, valine, leucine, isoleucine, proline, phenylalanine, methionine, tryptophan), β-branched side chain (e.g., threonine, valine, isoleucine), and aromatic side chain (e.g., tyrosine, phenylalanine, tryptophan, histidine). The conservative modifications may be selected, for example, based on polarity, charge, solubility, hydrophobicity, hydrophilicity, and/or similarity in amphiphilic properties of residues involved.

Thus, the "functional variant" or "functional fragment" has at least 75%, preferably at least 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the parent amino acid sequence, and retains the biological activity, e.g., binding activity, of the parent amino acid.

As used herein, the term "sequence identity" indicates the degree to which two (nucleotide or amino acid) sequences have the same residue at the same position in an alignment, and is generally expressed by percentage. Preferably, the identity is determined over the entire length of the sequences being compared. Thus, two copies with identical sequences have 100% identity. Those skilled in the art will recognize that some algorithms can be used to determine sequence identity using standard parameters, for example, Blast (Altschul et al. (1997) Nucleic Acids Res. 25:3389-3402), Blast2 (Altschul et al. (1990) J. Mol. Biol. 215:403-410), Smith-Waterman (Smith et al. (1981) J. Mol. Biol. 147:195-197), and ClustalW.

The selection of antigen-binding region depends on the cell surface marker on a target cell to be recognized and associated with a specific disease state, for example, a tumor specific antigen or a tumor associated antigen. Thus, in an embodiment, the antigen-binding region of the present invention binds to one or more targets selected from the group consisting of: TSHR, CD19, CD123, CD22, CD30, CD171, CS-1, CLL-1, CD33, EGFRvIII, GD2, GD3, BCMA, Tn Ag, PSMA, ROR1, FLT3, FAP, TAG72, CD38, CD44v6, CEA, EPCAM, B7H3, KIT, IL-13Ra2, mesothelin, IL-1 1Ra, PSCA, PRSS21, VEGFR2, LewisY, CD24, PDGFR-β, SSEA-4, CD20, Folate receptor α, ERBB2 (Her2/neu), MUC1, EGFR, NCAM, Prostase, PAP, ELF2M, Ephrin B2, IGF-I receptor, CAIX, LMP2, gp100, bcr-ab1, tyrosinase, EphA2, Fucosyl GM1, sLe, GM3, TGS5, HMWMAA, o-acetyl-GD2, Folate receptor β, TEM1/CD248, TEM7R, CLDN6, GPRC5D, CXORF61, CD97, CD 179a, ALK, polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, legumain, HPV E6, E7, MAGE A1, ETV6-AML, sperm protein 17, XAGE1, Tie 2, MAD-CT-1, MAD-CT-2, Fos associated antigen 1, p53, p53 mutant, prostate specific protein, survivin and telomerase, PCTA-1/Galectin 8, MelanA/MART1, Ras mutant, hTERT, sarcoma translocation breakpoint, ML-IAP, ERG (TMPRSS2 ETS fusion gene), NA17, PAX3, androgen receptor, Cyclin B1, MYCN, RhoC, TRP-2, CYP1B 1, BORIS, SART3, PAX5, OY-TES 1, LCK, AKAP-4, SSX2, RAGE-1, human telomerase reverse transcriptase, RU1, RU2, intestinal tract carboxylesterase, mut hsp70-2, CD79a, CD79b, CD72, LAIR1, FCAR, LILRA2, CD300LF, CLEC12A, BST2, EMR2, LY75, GPC3, FCRL5, IGLL1, PD1, PDL1, PDL2, TGF β, APRIL, NKG2D, and any combination thereof. Preferably, the target is selected from the group consisting of: CD19, CD20, CD22, BAFF-R, CD33, EGFRvIII, BCMA, GPRC5D, PSMA, ROR1, FAP, ERBB2 (Her2/neu), MUC1, EGFR, CAIX, WT1, NY-ESO-1, CD79a, CD79b, GPC3, Claudin18.2, NKG2D, and any combination thereof. Depending on the antigen to be targeted, the CAR of the present invention may be designed to include an antigen-binding region specific for the antigen. For example, if CD19 is the antigen to be targeted, a CD19 antibody can be used as an antigen-binding region of the present invention.

As used herein, the term "transmembrane domain" refers to a polypeptide structure that enables expression of a chimeric antigen receptor on the surface of an immune cell (e.g., a lymphocyte, an NK cell, or an NKT cell), and guides a cellular response of the immune cell against the target cell. The transmembrane domain may be natural or synthetic, and also may be derived from any membrane-bound protein or transmembrane protein. The transmembrane domain is capable of signaling when the chimeric receptor polypeptide binds to the target antigen. The transmembrane domains particularly suitable in the present invention may be derived from, for example, a TCR α chain, a TCR β chain, a TCR γ chain, a TCR δ chain, a CD3 ζ subunit, a CD3 ε subunit, a CD3 γ subunit, a CD3 δ subunit, CD45, CD4, CD5, CD8 α, CD9, CD16, CD22, CD33, CD28, CD37, CD64, CD80, CD86, CD134, CD137, CD154, and functional fragments thereof. Alternatively, the transmembrane domain may be synthesized and may mainly contain a hydrophobic residue such as leucine and valine. Preferably, the transmembrane domain is derived from a human CD8 α chain, which has at least 70%, preferably at least 80%, more preferably at least 90%, 95%, 97% or 99% or 100% sequence identity to an amino acid sequence represented by SEQ ID NO: 4 or a nucleotide sequence represented by SEQ ID NO: 3.

In an embodiment, the chimeric antigen receptor of the present invention further may contain a hinge region located between the antigen-binding region and the transmembrane domain. As used herein, the term "hinge region" generally refers to any oligopeptide or polypeptide that functions to link a transmembrane domain to an antigen-binding region. Specifically, the hinge region serves to provide greater flexibility and accessibility to the antigen-binding region. The hinge region may contain up to 300 amino acids, preferably 10 to 100 amino acids and most preferably 25 to 50 amino acids. The hinge region may be completely or partially derived from a natural molecule, for example, completely or partially from the extracellular region of CD8, CD4 or CD28, or completely or partially from an antibody constant region. Alternatively, the hinge region may be a synthetic sequence corresponding to a naturally occurring hinge sequence, or may be a completely synthetic hinge sequence. In a preferred embodiment, the hinge region contains a hinge region portion of a CD8 α chain, an Fc γ RIII α receptor, an IgG4, or an IgG1, more preferably a CD8 α hinge, which has at least 70%, preferably at least 80%, more preferably at least 90%, 95%, 97% or 99% or 100% sequence identity to an amino acid sequence represented by SEQ ID NO: 12 or a nucleotide sequence represented by SEQ ID NO: 11.

As used herein, the term "intracellular signaling domain" refers to a protein portion that transduces an effector function signal and guides a cell to perform a specified function. The intracellular signaling domain is responsible for intracellular primary signaling after the antigen-binding region binds to the antigen, thus causing activation of immune cell and immune reaction. In other words, the intracellular signaling domain is responsible for activating at least one of the normal effector functions of the immune cells in which the CAR is expressed. For example, the effector functions of T cell can be cytolytic activity or auxiliary activity, including secretion of cytokines.

The intracellular signaling domain contained in the chimeric antigen receptor of the present invention is the signaling domain of CD3 ζ.

Preferably, such signaling domain of CD3 ζ has at least 70%, preferably at least 80%, more preferably at least 90%, 95%, 97%, or 99% or 100% sequence identity to an amino acid sequence represented by SEQ ID NO: 8 or a nucleotide sequence represented by SEQ ID NO: 7.

The chimeric antigen receptor of the present invention contains one or more co-stimulatory signaling domains. The co-stimulatory signaling domain is the co-stimulatory signaling domain of CD137 (4-1BB). Preferably, the co-stimulatory domain of the CAR of the present invention has at least 70%, preferably at least 80%, more preferably at least 90%, 95%, 97% or 99% or 100% sequence identity to an amino acid sequence represented by SEQ ID NO: 6 or a nucleotide sequence represented by SEQ ID NO: 5.

In addition to the co-stimulatory domain and the intracellular signaling domain used for signaling, the chimeric antigen receptor of the present invention further contains at least one additional signaling region, wherein the additional signaling region consists of the intracellular region of the γc chain. In other words, the intracellular region (i.e., a structure for signaling) of the chimeric antigen receptor of the present invention consists of three signaling structures, namely, the co-stimulatory domain, the intracellular signaling domain, and the intracellular region of the γc chain. This means that the chimeric antigen receptor of the present invention does not contain a fourth signaling structure, for example, signaling regions of other cytokines, such as intracellular region of IL-2Ra, IL2Ra, IL2Rb, IL4Ra, IL7Ra, IL9Ra, IL15Ra, and IL21Ra.

As used herein, the term "γc chain" refers to the γ chain shared by the receptors of cytokines IL-2, IL-4, IL-7, IL-9, IL-15, and IL-21. The γc chain was initially identified in the IL-2 receptor, and it was later found that it is also involved in the composition of receptors such as IL-4, IL-7, IL-9, IL-15, and IL-21, so it is also called a common γ chain. For example, the IL-2 receptor has three forms, formed by different combinations of α chain (also known as IL-2Rα), β chain (also known as IL-2Rβ), and γc chain (also known as IL-2R γ or IL-2Rg), wherein compared with other combinations, the IL-2 receptor containing all three chains has the highest affinity to the IL-2 cytokine. The three chains of the IL-2 receptor are anchored to the cell membrane, and transmit a biochemical signal into the cell by binding to IL-2. In these γ chain-dependent cytokines, cytokine receptor-specific components, such as IL-2Rβ, IL-4Rα, IL-7Rα, IL-9Rα, and IL-21R, are responsible for binding to JAK1, while the γc chain binds to JAK3. When these cytokine receptors bind to cytokines, three major signaling pathways are activated, including MAP kinase, PI3 kinase, and JAK-STAT pathway, thus regulating the survival and proliferation of T cell and NK cell.

γc chain is a glycoprotein having a molecular weight of 64 kD, and consists of 347 amino acids, in which an extracellular region of 232 amino acids, a transmembrane region of 29 amino acids, and an intracellular region of 86 amino acids are included. The intracellular region contains an Src homologous region, which is crucial for promoting the growth of cells and the expression of IL-2-mediated genes such as c-myc, c-fos, and c-jun. In an embodiment, the intracellular region of the γc chain is represented by SEQ ID NO: 16 or a nucleotide sequence represented by SEQ ID NO: 15. Preferably, the intracellular region of the γc chain consists of SEQ ID NO: 16.

In an embodiment, the CAR of the present invention further may contain a signal peptide such that when it is expressed in a cell such as a T cell, the nascent protein is directed to the endoplasmic reticulum and subsequently to the cell surface. The core of the signal peptide may contain a long hydrophobic amino acid segment, which has a tendency to form a single α-helix. At the end of the signal peptide, there is usually an amino acid segment recognized and cleaved by signal peptidase. The signal peptidase can cleave during or after translocation, so as to generate free signal peptide and mature protein. Then, the free signal peptide is digested by a specific protease. Signal peptides that can be used in the present invention are well known to those skilled in the art, for example, signal peptides derived from CD8 α, IgG1, and GM-CSFRα.

In a preferred embodiment, the chimeric antigen receptor of the present invention includes a CD8 α transmembrane domain, a 4-1BB co-stimulatory domain, a CD3 ζ signaling domain, and an intracellular region of the γc chain. More preferably, the chimeric antigen receptor further contains a CD8 α signal peptide, a CD8 α hinge region, and/or a CD28 co-stimulatory domain.

The chimeric antigen receptor of the present invention, the co-stimulatory domain, the intracellular signaling domain, and the additional signaling region are arranged in order of distance from the cell membrane from nearest to farthest, that is, the co-stimulatory domain is closest to the cell membrane, while the additional signaling region is furthest away from the cell membrane.

### Nucleic acid

The present invention further provides a nucleic acid, which contains a sequence encoding the chimeric antigen receptor of the present invention.

As used herein, the term "nucleic acid" includes a sequence of ribonucleotides and deoxyribonucleotides, such as modified or unmodified RNA or DNA, each in single-stranded and/or double-stranded form, linear or circular, or their mixtures (including hybrid molecules). Thus, the nucleic acid according to the present invention includes DNA (e.g. dsDNA, ssDNA, cDNA), RNA (e.g. dsRNA, ssRNA, mRNA, ivtRNA), their combinations or derivatives (e.g. PNA). Preferably, the nucleic acid is DNA or RNA, more preferably mRNA.

The nucleic acid may contain a conventional phosphodiester bond or an unconventional bonds (e.g., amide bond, such as found in peptide nucleic acid (PNA)). The nucleic acid of the present invention further may contain one or more modified bases, such as, for example, trityl base and uncommon base (such as inosine). Other modifications also can be contemplated, including chemical, enzymatic, or metabolic modifications, so long as the multi-chain CAR of the present invention can be expressed from polynucleotides. The nucleic acid can be provided in isolated form. In an embodiment, the nucleic acid also may include a regulatory sequence, such as a transcriptional control element (including a promoter, an enhancer, an operon, a repressor, and a transcription termination signal), ribosome binding sites, and introns.

The nucleic acid sequences of the present invention can be codon-optimized for optimal expression in a desired host cell (e.g., immune cell); or for expression in a bacterial, yeast, or insect cell. Codon optimization refers to substitution of a codon in the target sequence that is generally rare in highly expressed genes of a given species with a codon that is generally common in highly expressed genes of such species, and the codons before and after the substitution encode the same amino acid. Therefore, the selection of an optimal codon depends on the codon usage preference of the host genome.

### Vector

The present invention further provides a vector, containing one or more nucleic acids as described in the present invention.

As used herein, the term "vector" is a nucleic acid molecule used as an intermediary to transfer (exogenous) genetic material into a host cell, wherein in the host cell the nucleic acid molecule can be, for example, replicated and/or expressed.

The vector generally includes targeting vectors and expression vectors. The "targeting vector" is a medium that delivers an isolated nucleic acid to the interior of a cell by, for example, homologous recombination or by using a hybrid recombinases of a sequence at specific target site. The "expression vector" is a vector used for the transcription of heterologous nucleic acid sequences (for example, those sequences encoding the chimeric antigen receptor polypeptides of the present invention) in suitable host cells and the translation of their mRNAs. Suitable vectors that can be used in the present invention are known in the art, and many are commercially available. In an embodiment, the vectors of the present invention include, but are not limited to, linear nucleic acid molecule (e.g., DNA or RNA), plasmid, virus (e.g., retrovirus, lentivirus, adenovirus, vaccinia virus, Rous sarcoma virus (RSV), multiple tumor virus, and adeno-associated virus (AAV), etc.), bacteriophage, phagemid, cosmid, and artificial chromosome (including BAC and YAC). The vector itself is usually a nucleotide sequence, and usually is a DNA sequence containing the insert (transgene) and a larger sequence acting as "backbone" of the vector. Engineered vector typically also contains an origin autonomously replicating in the host cell (if stable expression of the polynucleotide is desired), a selectable marker, and a restriction enzyme cleavage site (e.g., a multiple cloning site, MCS). The vectors may additionally contain elements such as a promoter, a poly-A tail (polyA), 3' UTR, an enhancer, a terminator, an insulator, an operon, a selectable marker, a reporter gene, a targeting sequence, and/or a protein purification tag. In a specific embodiment, the vector is an *in vitro* transcription vector. In a specific embodiment, the vector is an *in vitro* transcription vector.

### Engineered immune cell and preparation method thereof

The present invention provides an engineered immune cell, which contains a chimeric antigen receptor or a nucleic acid encoding the same.

As used herein, the term "immune cell" refers to any cell of the immune system that has one or more effector functions (e.g., cytotoxic cell killing activity, secretion of cytokines, induction of ADCC and/or CDC). For example, the immune cell may be a T cell, a macrophage, a dendritic cell, a monocyte, an NK cell, and/or an NKT cell. Preferably, the immune cell is a T cell. The T cell may be any T cell, such as *in vitro* cultured T cell, for example, primary T cell, or T cell from *in vitro* cultured T cell line, e.g., Jurkat, SupT1, etc., or T cell obtained from a subject. Examples of subject include humans, dogs, cats, mice, rats, and transgenic species thereof. The T cell can be obtained from a variety of sources, including peripheral blood monocytes, bone marrow, lymph node tissue, umbilical blood, thymus tissue, tissue from sites of infection, ascites, pleural effusion, spleen tissue, and tumors. The T cell also may be concentrated or purified. The T cell may be any type of T cell and may be at any stage of development including, but not limited to, a CD4+/CD8+ double positive T cell, a CD4+ helper T cell (e.g., Th1 and Th2 cells), CD8+ T cell (e.g., cytotoxic T cell), tumor infiltrating cell, memory T cell, naive T cell, γδ-T cell, αβ-T cell, etc. In a preferred embodiment, the immune cell is a human T cell. The T cell can be isolated from the blood of a subject using a variety of techniques known to those of skill in the art, such as Ficoll. In the present invention, the immune cell is engineered to express the chimeric antigen receptor polypeptides.

The nucleic acid sequence encoding the chimeric antigen receptor polypeptide can be introduced into the immune cell to express the chimeric antigen receptor polypeptide of the present invention using conventional methods known in the art (e.g., by transduction, transfection, transformation, etc.). "Transfection" is a process of introducing a nucleic acid molecule or polynucleotide (including a vector) into a target cell. An example is RNA transfection, i.e., the process of introducing RNA (such as *in vitro* transcribed RNA, ivtRNA) into a host cell. This term is primarily used for a non-viral method in eukaryotic cells. The term "transduction" is generally used to describe virusmediated transfer of nucleic acid molecules or polynucleotides. Transfection of animal cells typically involves opening transient pores or "holes" in the cell membrane, so as to allow uptake of material. Transfection may be carried out using calcium phosphate, by electroporation, by extrusion of cells, or by mixing cationic lipids with the material so as to produce liposomes which fuse with the cell membrane and deposit their cargo into the interior. Exemplary techniques for transfecting eukaryotic host cells include lipid vesicle-mediated uptake, heat shock-mediated uptake, calcium phosphate-mediated transfection (calcium phosphate/DNA co-precipitation), microinjection, and electroporation. The term "transformation" is used to describe the non-virus transfer of a nucleic acid molecule or polynucleotide (including a vector) to bacteria, and also to non-animal eukaryotic cells (including plant cells). Thus, the transformation is a genetic alteration of bacterial or non-animal eukaryotic cells, which is produced by direct uptake of a cell membrane from its surroundings and subsequent incorporation of exogenous genetic material (nucleic acid molecule). The transformation can be achieved by artificial means. In order for transformation to occur, the cell or bacterium must be in a competent state. For prokaryotic transformation, the techniques may include heat-shock-mediated uptake, fusion to bacterial protoplasts of intact cells, microinjection, and electroporation.

Still in an embodiment, the immune cell of the present invention further contains at least one inactive gene selected from the group consisting of CD52, GR, TCR α, TCR β, CD3 γ, CD3 δ, CD3 ε, CD247 ζ, HLA-I, HLA-II gene, and immune checkpoint gene such as PD1 and CTLA-4. More particularly, at least the TCR α or TCR β gene in the immune cell is inactivated. Such inactivation renders the TCR non-functional in cells. This strategy is particularly useful for avoiding graft-versus-host disease (GvHD). Methods for inactivating a gene are known in the art, for example, by mediating DNA breakage by a meganuclease, a zincfinger nuclease, a TALE nuclease, or a Cas enzyme in a CRISPR system, thereby inactivating the gene.

### Pharmaceutical composition

The present invention further provides a pharmaceutical composition, which contains the chimeric antigen receptor, the nucleic acid, the vector or the engineered immune cell of the present invention as an active agent, and one or more pharmaceutically acceptable excipients. Therefore, the present invention further encompasses use of the chimeric antigen receptor, nucleic acid, vector or engineered immune cell in the preparation of a pharmaceutical composition or medicine.

As used herein, the term "pharmaceutically acceptable excipient" refers to a vector and/or excipient that is pharmacologically and/or physiologically compatible (i.e., capable of triggering a desired therapeutic effect without causing any undesired local or systemic effects) with the subject and active ingredient, which is well known in the art (see, e.g., Remington's Pharmaceutical Sciences. Edited by Gennaro AR, 19th ed. Pennsylvania: Mack Publishing Company, 1995). Examples of pharmaceutically acceptable excipient include, but are not limited to, filler, binder, disintegrant, coating agent, adsorbent, anti-adherent, glidant, antioxidant, flavoring agent, colorant, sweetener, solvent, co-solvent, buffer agent, chelating agent, surfactant, diluent, wetting agent, preservative, emulsifier, cladding agent, isotonic agent, absorption delaying agent, stabilizer, and tension regulator. It is known to those skilled in the art to select a suitable excipient to prepare the desired pharmaceutical composition of the present invention. Exemplary excipients for use in the pharmaceutical compositions of the present invention include saline, buffered saline, dextrose, and water. Generally, the selection of a suitable excipient depends, in particular, on the active agent used, the disease to be treated, and the desired dosage form of the pharmaceutical composition.

The pharmaceutical composition according to the present invention is suitable for multiple routes of administration. Generally, the administration is parenterally accomplished. Parenteral delivery methods include topical, intraarterial, intramuscular, subcutaneous, intramedullary, intrathecal, intraventricular, intravenous, intraperitoneal, intrauterine, intravaginal, sublingual, or intranasal administration.

The pharmaceutical composition according to the present invention also can be prepared in various forms, such as solid, liquid, gaseous or lyophilized forms, particularly the pharmaceutical composition can be prepared in the form of ointment, cream, transdermal patch, gel, powder, tablet, solution, aerosol, granule, pill, suspension, emulsion, capsule, syrup, elixir, extract, tincture or liquid extract, or in a form particularly suitable for the desired method of administration. Processes known for producing a medicine may include, for example, conventional mixing, dissolving, granulating, dragee-making, grinding, emulsifying, encapsulating, embedding or lyophilizing process. The pharmaceutical composition containing, for example, the immune cell as described herein is generally provided in a form of solution, and preferably contains a pharmaceutically acceptable buffer agent.

The pharmaceutical composition according to the present invention further may be administered in combination with one or more other agents suitable for the treatment and/or prophylaxis of diseases to be treated. Preferred examples of agent suitable for the combination include known anti-cancer medicines such as cisplatin, maytansine derivatives, rachelmycin, calicheamicin, docetaxel, etoposide, gemcitabine, ifosfamide, irinotecan, melphalan, mitoxantrone, sorfimer sodiumphotofrin II, temozolomide, topotecan, trimetreate glucuronate, auristatin E, vincristine and doxorubicin; peptide cytotoxins, such as ricin, diphtheria toxin, pseudomonas exotoxin A, DNase and RNase; radionuclides such as iodine 131, rhenium 186, indium 111, iridium 90, bismuth 210 and 213, actinide 225 and astatine 213; prodrugs such as antibody-directed enzyme prodrugs; immunostimulatory agents such as IL-2, chemokines such as IL-8, platelet factor 4, and melanoma growth stimulating protein; antibodies or fragments thereof, such as anti-CD3 antibodies or fragments thereof, complement activators, heterologous protein domains, homologous protein domains, viral/bacterial protein domains and viral/bacterial peptides. In addition, the pharmaceutical composition of the present invention can be used in combination with one or more other treatment methods, such as chemotherapy and radiotherapy.

### Method of preparing engineered immune cell

A method of preparing an engineered immune cell of the invention is also disclosed. The method includes introducing the chimeric antigen receptor or the nucleic acid sequence encoding the same of the present invention into an immune cell, so that the immune cell expresses the chimeric antigen receptor of the present invention.

In an embodiment, the immune cell is a human immune cell, more preferably a human T cell, a macrophage, a dendritic cell, a monocyte, an NK cell, and/or an NKT cell.

Methods of introducing a nucleic acid or vector into an immune cell and expressing the same are known in the art. For example, the nucleic acid or vector can be introduced into the immune cell by a physical method, such as calcium phosphate precipitation, lipofection, particle bombardment, microinjection, and electroporation. Alternatively, a chemical method also can be employed, for example, the nucleic acid or vector is introduced by a colloid dispersion system such as macromolecular complex, nanocapsule, microsphere, bead, and a lipid-based system, including oil-in-water emulsion, micelle, mixed micelle, and liposome. In addition, a biological method also can be used to introduce the nucleic acid or vector. For example, a viral vector, particularly a retroviral vector, and the like have become the most commonly used method of inserting a gene into a mammal, such as human, cell. Other viral vectors may be derived from lentivirus, poxvirus, herpes simplex virus I, adenovirus, and adeno-associated virus, etc.

After introducing the nucleic acid or vector into the immune cell, a person skilled in the art could amplify and activate the resulting immune cell by conventional techniques.

### Therapeutic application

The present invention further provides a medical indicationfor treating a subject with cancer, including administering to the subject an effective amount of the immune cell or the pharmaceutical composition according to the present invention.

In an embodiment, an effective amount of the immune cell and/or the pharmaceutical composition of the present invention is directly administered to the subject.

In another embodiment, the immune cell and/or the pharmaceutical composition is administered *ex vivo.* Specifically, this embodiment includes the steps of: (a) providing a sample of a subject, the sample containing an immune cell; (b) introducing the chimeric antigen receptor of the present invention into the immune cell *in vitro* to obtain a modified immune cell, and (c) administering the modified immune cell to the subject in need thereof. Preferably, the immune cell provided in step (a) is selected from a T cell, an NK cell, and/or an NKT cell; and the immune cell can be obtained from the sample (particularly a blood sample) of the subject by a conventional method known in the art. However, other immune cells capable of expressing the chimeric antigen receptor of the present invention and exerting the desired biological effect function as described herein also can be used. Besides, the immune cells generally selected are compatible with the subject's immune system, i.e., it is preferred that the immune cells do not trigger an immunogenic response. For example, a "universal recipient cell", i.e., a universally compatible lymphocyte exerting a desired biological effect function and being capable of growing and amplifying *in vitro,* can be used. The use of such cells will not require obtaining and/or providing the subject's own lymphocyte. The *ex vivo* introduction of step (c) may be carried out by introducing the nucleic acid or vector described herein into the immune cell via electroporation or by infecting the immune cell with a viral vector, wherein the viral vector is a lentiviral vector, adenoviral vector, adeno-associated viral vector or retroviral vector as previously described. Other conceivable methods include using a transfection reagent (such as a liposome) or transient RNA transfection.

In an embodiment, the immune cell is an autologous or allogeneic cell, preferably T cell, macrophage, dendritic cell, monocyte, NK cell and/or NKT cell, more preferably T cell, NK cell or NKT cell.

As used herein, the term "autologous" means that any material derived from an individual will be later re-introduced into the same individual.

As used herein, the term "allogeneic" means that the material is derived from a different animal or different patient of the same species as the individual into which the material is introduced. When the genes at one or more loci are different, two or more individuals are considered allogeneic to each other. In some cases, genetic differences in allogeneic material from various individuals of the same species may be sufficient for antigen interactions to occur.

As used herein, the term "subject" refers to a mammal. The mammal may be, but is not limited to, a human, a non-human primate, a mouse, a rat, a dog, a cat, a horse, or a cow. Mammals other than human can be advantageously used as subjects representing cancer animal models. Preferably, the subject is a human.

In an embodiment, the disease is a cancer associated with expression of the target to which the antigen-binding region binds. For example, the cancer includes, but is not limited to, brain glioma, blastoma, sarcoma, leukemia, basal cell carcinoma, biliary tract cancer, bladder cancer, bone cancer, brain and CNS cancer, breast cancer, peritoneal cancer, cervical cancer, choriocarcinoma, colon and rectal cancer, connective tissue cancer, cancer of digestive system, endometrial cancer, esophageal cancer, eye cancer, head and neck cancer, stomach cancer (including gastrointestinal cancer), glioblastoma (GBM), liver cancer, hepatoma, intraepithelial tumor, kidney cancer, larynx cancer, liver tumor, lung cancer (such as small cell lung cancer, non-small cell lung cancer, lung adenocarcinoma and squamous lung cancer), lymphoma (including Hodgkin's lymphoma and non-Hodgkin's lymphoma), melanoma, myeloma, neuroblastoma, oral cancer (e.g., lips, tongue, mouth, and pharynx), ovarian cancer, pancreatic cancer, prostate cancer, retinoblastoma, rhabdomyosarcoma, rectal cancer, cancer of respiratory system, salivary gland cancer, skin cancer, squamous cell carcinoma, stomach cancer, testicular cancer, thyroid cancer, uterine or endometrial cancer, malignant tumor of urinary system, vulval cancer and other cancers and sarcomas, and B cell lymphoma (including low-grade/follicular non-Hodgkin's lymphoma (NHL), small lymphocytic (SL) NHL, intermediate-grade/follicular NHL, intermediate-grade diffuse NHL, high-grade immunoblastic NHL, high-grade lymphoblastic NHL, high-grade small non-cracked cell NHL, bulky disease NHL), mantle cell lymphoma, AIDS-related lymphoma, and Waldenstrom macroglobulinemia, chronic lymphocytic leukemia (CLL), acute lymphocytic leukemia (ALL), B cell acute lymphocytic leukemia (B-ALL), T cell acute lymphocytic leukemia (T-ALL), B cell prolymphocytic leukemia, blast cell plasmacytoid dendritic cell tumor, Burkitt lymphoma, diffuse large B cell lymphoma, follicular lymphoma, chronic myelogenous leukemia (CML), malignant lymphoproliferative disorder, MALT lymphoma, hairy cell leukemia, marginal zone lymphoma, multiple myeloma, myelodysplasia, plasmablastic lymphoma, preleukemia, plasmacytoid dendritic cell tumor, post-transplant lymphoproliferative disorder (PTLD), and other diseases associated with target expression. Preferably, the disease which can be treated with the engineered immune cell or the pharmaceutical composition of the present invention is selected from the group consisting of: leukemia, lymphoma, multiple myeloma, brain glioma, pancreatic cancer, gastric cancer, and so on.

In an embodiment, the subject is further administered one or more additional chemotherapeutic agents, biological agents, medicines, or treatments. In this embodiment, the chemotherapeutic agents, biological agents, medicines, or treatments is selected from the group consisting of radiotherapy, surgery, antibody reagent and/or small molecule and any combination thereof.

The present invention will be described in detail below with reference to the accompanying drawings and examples. The examples of the present disclosure and the features in the examples may be combined with each other without contradiction.

### Brief Description of Drawings

FIG. 1: CAR expression levels of CAR-T cells detected by flow cytometry.
FIG. 2: Killing effect of CAR-T cells on target cells. Two-way ANOVA is used for analysis, and T test is used for statistical analysis. * indicates that P value is smaller than 0.05, reaching a significant level.
FIG. 3: IL-2 (A) and IFN γ (B) release levels after co-culture of CAR-T cells with target cells and non-target cells, respectively.
FIG. 4: Changes of tumor burden in treated mice over time.
FIG. 5: Amplification levels of CD3+ (A), CD8+ (B), and CD4+ (C) T cells in mice on day D21.
FIG. 6: Changes of survival rate of treated mice over time.
FIG. 7: Killing effect of CAR-T cells on target cells. Two-way ANOVA is used for analysis, and T test is used for statistical analysis. * indicates that P value is smaller than 0.05, reaching a significant level.

### Detailed Description of Embodiments

Sequences used in the following examples are summarized as in Table 1 below.

**Table 1. Sequences used in the present disclosure**

| SEQ ID NO | Description |
|---|---|
| SEQ ID NO: 1 | nucleotide sequence of CD19-scFv |
| SEQ ID NO: 2 | amino acid sequence of CD19-scFv |
| SEQ ID NO: 3 | nucleotide sequence of transmembrane domain CD8 α |
| SEQ ID NO: 4 | amino acid sequence of transmembrane domain CD8 a |
| SEQ ID NO: 5 | nucleotide sequence of co-activation domain 4-1BB |
| SEQ ID NO: 6 | amino acid sequence of co-activation domain 4-1BB |
| SEQ ID NO: 7 | nucleotide sequence of signaling domain CD3 ζ |
| SEQ ID NO: 8 | amino acid sequence of signaling domain CD3 ζ |
| SEQ ID NO: 9 | nucleotide sequence of signal peptide CD8 α |
| SEQ ID NO: 10 | amino acid sequence of signal peptide CD8 α |
| SEQ ID NO: 11 | nucleotide sequence of CD8 α hinge region |
| SEQ ID NO: 12 | amino acid sequence of CD8 a hinge region |
| SEQ ID NO: 13 | nucleotide sequence of γc chain |
| SEQ ID NO: 14 | amino acid sequence of γc chain |
| SEQ ID NO: 15 | nucleotide sequence of γc chain intracellular region |
| SEQ ID NO: 16 | amino acid sequence of γc chain intracellular region |

The T cells used in all the examples of the present disclosure are primary human CD4+CD8+ T cells isolated from healthy donors by Ficoll-PaqueTM PREMIUM (GE Healthcare, Article No. 17-5442-02) using leukapheresis. Nalm6 tumor cells were purchased from GemPharmatech, Co. Ltd.

### Example 1: Construction of CAR T cell

The following encoding sequences were synthesized, and sequentially cloned into pGEM-T Easy vector (Promega, Lot No. A1360): CD8 α signal peptide (SEQ ID NO: 9), anti-CD19 scFv (SEQ ID NO: 1), CD8 α hinge region (SEQ ID NO: 11), CD8 α transmembrane region (SEQ ID NO: 3), 4-1BB co-stimulatory domain (SEQ ID NO: 5), CD3 ζ intracellular signaling domain (SEQ ID NO: 7), to obtain a conventional bbz-CAR plasmid, and correct insertion of the target sequence was confirmed by sequencing. A bbzg-CAR plasmid was obtained in the same way, which differs from the bbz-CAR plasmid only in that it further includes a γ chain intracellular region (SEQ ID NO: 15) linked to the CD3 ζ intracellular signaling domain. In the bbzg-CAR plasmid, the 4-1BB co-stimulatory domain, the CD3 ζ intracellular signaling domain, and the γ chain intracellular region are arranged in order of distance from the cell membrane from nearest to farthest.

After 3 ml of Opti-MEM (Gibco, Lot No. 31985-070) was added to a sterile tube to dilute the above plasmids, a packaging vector psPAX2 (Addgene, Lot No. 12260) and an envelope vector pMD2. G (Addgene, Lot No. 12259) were then added according to a ratio of plasmid: virus packaging vector: virus envelope vector = 4:2:1. Then, 120 µl X-treme GENE HP DNA transfection reagent (Roche, Lot No. 06366236001) was added, well mixed immediately, followed by incubation at room temperature for 15 min, and then the plasmid/vector/transfection reagent mixture was added dropwise into a culture flask of 293T cells. The viruses were collected at 24 and 48 hours and combined, and then subjected to ultracentrifugation (25000 g, 4 °C, 2.5 hours) to obtain a concentrated lentivirus.

T cells were activated with DynaBeads CD3/CD28 CTSTM (Gibco, Lot No. 40203D), and cultured for 1 day at 37 °C and 5% CO₂. Then, the concentrated lentivirus was added, and after 3 days of continuous culture, the conventional con-CAR T cells targeting CD19 (used as control) and the bbzg-CAR T cells of the present invention were obtained.

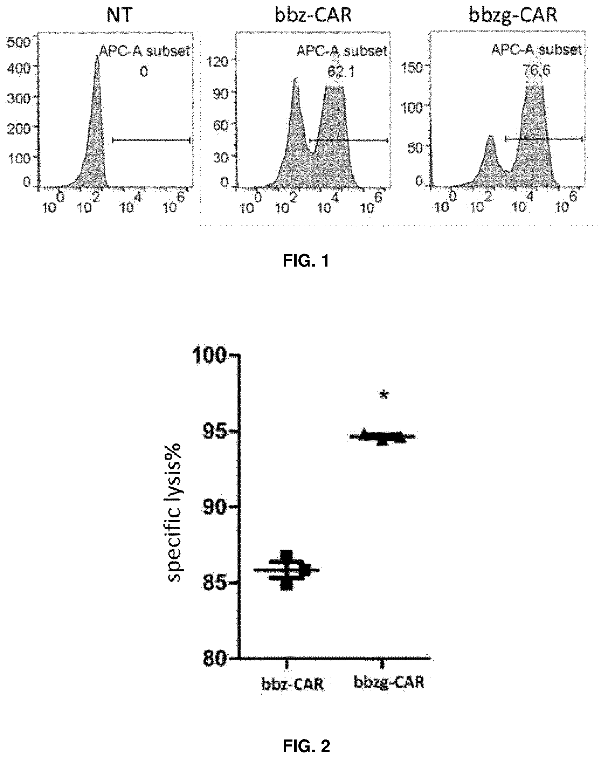

After 11 days of culture at 37 °C and 5% CO₂, expression levels of scFv on Fite-CAR T cells were detected by flow cytometry with Biotin-SP (long spacer) AffiniPure Goat Anti-Mouse IgG, F(ab')₂ Fragment Specific (min X Hu, Bov, Hrs Sr Prot) (jackson immunoresearch, Lot No. 115-065-072) as a primary antibody, and APC Streptavidin (BD Pharmingen, Lot No. 554067) or PE Streptavidin (BD Pharmingen, Lot No. 554061) as a secondary antibody, and the results are as shown in FIG. 1 (NT is unmodified wild-type T cell).

It can be seen that the bbzg-CAR T cell of the present invention can effectively express the scFv, and the expression level thereof is slightly higher than that of the conventional bbz-CAR T cell, indicating that the addition of the γ chain intracellular region does not affect the surface expression of the CAR structure.

### Example 2: Killing effect of CAR T cell on target cells and cytokine release

### 2.1 Killing effect of CAR-T cell on target cells

When T cell kills target cells, the number of target cells is reduced. After T cell and target cells capable of expressing luciferase are co-cultured, the number of target cells is reduced, and meanwhile, secreted luciferase is also reduced therewith. Luciferase can catalyze conversion of fluorescein into oxidating fluorescein, and during this oxidation, bioluminescence is generated. The intensity of such luminescence depends on the level of luciferase expressed by the target cells. Thus, the detected fluorescence intensity can reflect the killing ability of T cell to the target cells.

In order to detect the killing ability of CAR-T cell to the target cells, Nalm6 target cells carrying a fluorescein gene were first plated into a 96-well plate at 1×10⁴ per well, then bbzg-CAR T cells, Con-CAR T cells (positive control), and untransfected T cells (negative control) were plated into the 96-well plate at an effector-target ratio of 32:1 (i.e. a ratio of effector T cells to target cells) for co-culture, and a fluorescence value was measured with a plate reader 16-18 hours later. According to the calculation formula: (mean value of fluorescence of target cell - mean value of fluorescence of sample)/mean value of fluorescence of target cell × 100%, the killing efficiency was calculated, and the result is as shown in FIG. 2.

It can be seen that compared with NT, the killing effect of the bbzg-CAR T cell of the present invention on target cells is significantly higher than that of the conventional bbz-CAR T cell.

### 2.2 Cytokine release of CAR-T cell

When the T cell kills the target cells, cytokines IL2, IFN-γ, etc. are also released while the number of target cells is reduced. The release levels of cytokines IL2 and IFN γ when the Fite-CARX T cell killed the target cells were detected by enzyme-linked immunosorbent assay (ELISA) according to the following steps.

### (1) Collecting cell co-culture supernatant

Target cells (Nalm6 and Raji) and non-target cells (193F) were plated in a 96-well plate at 1×10⁵/well, respectively, then the bbzg-CAR T, con-CAR T (positive control), and NT cells (negative control) were co-cultured with target or non-target cells, respectively, at a ratio of 1:1, and cell co-culture supernatant was collected 18-24 hours later.

### (2) Detection of IFN γ secretion in supernatant by ELISA

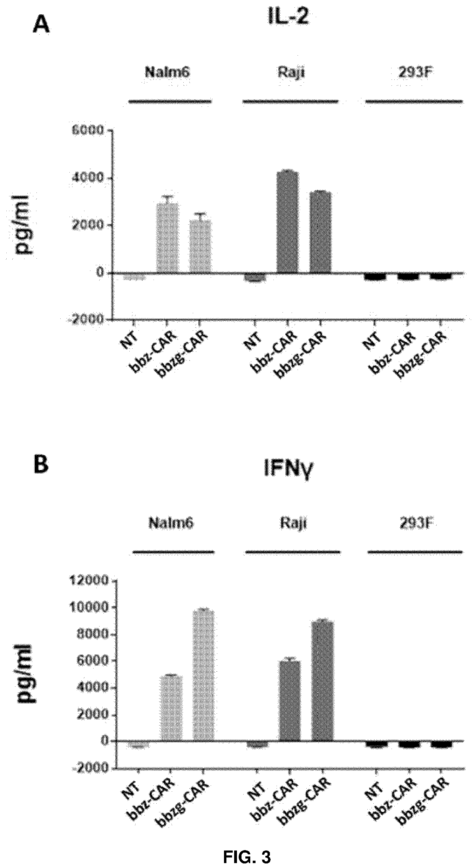

The 96-well plate was coated with capture antibody Purified anti-human IFN-γ Antibody (Biolegend, Article No. 506502) and incubated overnight at 4 °C, then the antibody solution was removed, 250 µL of PBST (IXPBS containing 0.1% Tween) solution containing 2% BSA (sigma, Article No. V9009333-1 kg) was added, followed by incubation at 37 °C for 2 hours. The plate was then washed 3 times with 250 µL of PBST (1XPBS containing 0.1% Tween). 50 µL of cell co-culture supernatant or standard was added to each well, followed by incubation at 37 °C for 1 hour, then the plate was washed 3 times with 250 µL of PBST (1XPBS containing 0.1% Tween). Then 50 µL of detection antibody Anti-Interferon gamma antibody [MD-1] (Biotin) (abcam, Article No. ab25017) was added to each well, and after 1 hour of incubation at 37 °C, the plate was washed 3 times with 250 µL of PBST (1XPBS containing 0.1% Tween). HRP Streptavidin (Biolegend, Article No. 405210) was then added, and after 30 minutes of incubation at 37 °C, the supernatant was discarded, 250 µL of PBST (1XPBS containing 0.1% Tween) was added, and the plate was washed 5 times. 50 µL of TMB substrate solution was added to each well. The reaction was carried out in the dark at room temperature for 30 minutes, after which 50 µL of 1 mol/L H₂ SO₄ was added to each well to stop the reaction. Within 30 minutes after the stop of the reaction, the absorbance at 450 nm was detected by a plate reader, and the content of cytokines was calculated according to a standard curve (drawn according to read value and concentration of the standard), and the result is as shown in FIG. 3.

It can be seen that no release of IFN γ was detected in non-target cells 293F, indicating that killing of both bbz-CAR T cells and bbzg-CAR T cells is specific. Furthermore, when killing the target cells, the bbzg-CAR T cells has a significantly lower IL2 release level than the conventional CAR T cells, but a significantly higher IFN-γ release level than the conventional CAR T cells. On the whole, the cytokine release of the bbzg-CAR T cells of the present invention is comparable to the conventional CAR-T cells.

### Example 3 Verification of tumor inhibition effect of CAR-T cell

The inhibitory effect of CAR-T cell on tumors was verified in mouse models.
20 8-week healthy female NCG mice were divided into four groups: PBS group, NT group (negative control), bbz-CART group (positive control), and bbzg-CAR T group. 1×10⁶ Nalm6 cells were injected into each mouse through tail vein on day 0 (D0). 7 days (D7) later, PBS solution or 2 × 10⁶ NT cells, con-CAR T cells or bbzg-CAR T cells were injected into each mouse through tail vein according to the grouping. Mice were assessed weekly for changes in survival rate and tumor burden.

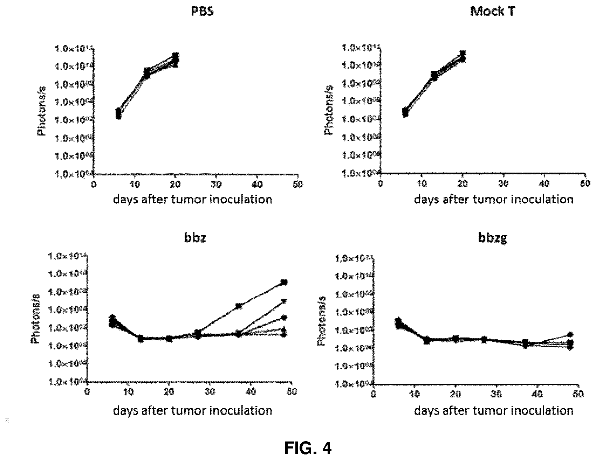

Tumor burden changes in each group of mice were assessed by optical *in vivo* imaging technologies for living animals. Mouse tumor burden was detected on D7, D14, D21, D28, D35, D42, and D49 and was expressed in Photons/s, with the results as shown in FIG. 4.

It can be seen that in the PBS and NT groups, the tumor burden in mice progressed rapidly, and reached the highest value on D21 (immediately died). For the mice of the bbz-CAR group, the tumor burden rapidly decreased after receiving the CAR-T cell treatment, but gradually rebounded on D28 or D35. In contrast, tumor burden of the mice in the bbzg-CAR T group not only rapidly decreased after receiving the treatment, but also maintained a low level until D49 without recurrence. This indicates that the bbzg-CAR T cell of the present invention can effectively inhibit the tumor growth, and the effect is significantly superior to that of the conventional bbz-CAR T cell.

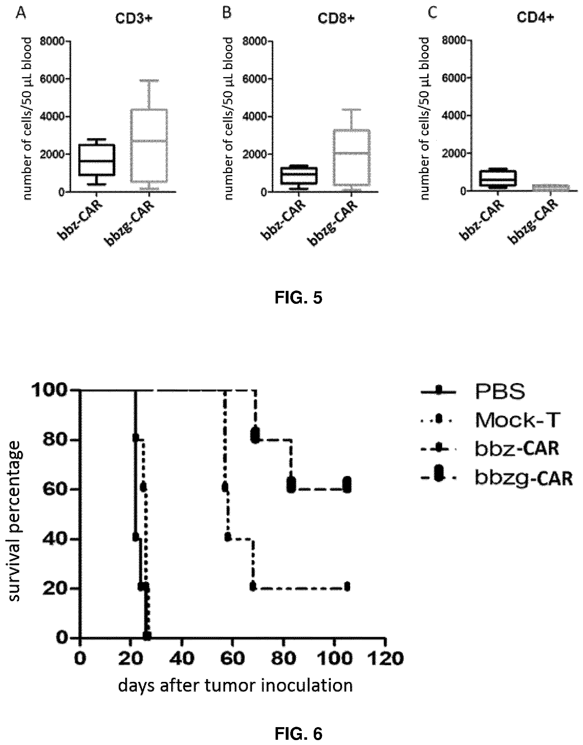

The inventors also monitored the T cell amplification of two groups of mice receiving the treatment of bbz-CART cell and bbzg-CAR T cell on day 21. Specifically, blood was taken from submandibular vein of the mice on D21, and subjected to Trucount FACS analysis (expression levels of hCD3, hCD8, hCD4), and the results are as shown in FIG. 5.

It can be seen that T cell amplification is detected in both the bbz-CAR T group and the bbzg-CAR T group of mice. Although the amplification extents of the CD4+ T cells in the two groups are comparable, the amplification of CD3+ and CD8+ T cells in the bbzg-CAR group is significantly higher than that in the bbz-CAR T group. Therefore, although the tumor burden of the two groups on day 21 is almost the same (FIG. 4), as the T cell amplification in the mice of the bbzg-CAR T group is significantly more, the tumor burden can constantly be inhibited at a lower level; in contrast, due to less amplification and continued depletion of T cells, the tumor burden of the mice in the bbz-CAR T group rebounds afterwards.

In addition, the inventors also counted the survival percentage of each group of mice by the end of the experiment (namely, day 105 after inoculation of tumor cells Nalm6) (FIG. 6). In the above, all of the mice in the PBS and NT groups died, only one (20%) mouse treated with the bbz-CAR T cells survived, while 60% of the mice treated with the bbzg-CAR T cells survived. This again indicates that the bbzg-CAR T cell of the present invention can effectively inhibit tumors and improve the survival rate.

To sum up, compared with the conventional CAR T cells, the bbzg-CAR T cell of the present invention, due to the introduction of the cytokine receptor general γ chain, can greatly promote the amplification of the T cell, thus improving the sustained killing effect on tumor cells, improving the *in vivo* tumor inhibitory effect, and increasing the survival of mice.

### Example 4. Preparation of CAR-T cells with different structures and verification of functions thereof

The γc chain intracellular region (SEQ ID NO: 15) was inserted between the 4-1BB co-stimulatory domain and the CD3 ζ primary signaling domain of the bbz-CAR plasmid to obtain the bbgz-CAR plasmid, which differs from the bbzg-CAR plasmid only in that the position of the γc chain intracellular region is different. The bbgz-CAR T cell was prepared according to the method of Example 1.

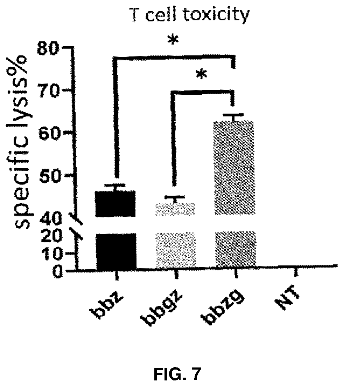

The killing effect of CAR-T cell on target cells was detected according to the method described in Example 2, and the results are as shown in FIG. 7. It can be seen that the killing effect of the bbgz-CAR T cell on target cells is comparable to that of the conventional bbz-CAR T cell, but both are significantly lower than the killing effect of the bbzg-CAR T cell. This indicates that the position of the additional signaling region (i.e., the intracellular region of the γc chain) in the CAR structure has an important influence on the killing activity of CAR T cell.

## Claims

1. A chimeric antigen receptor, containing an antigen-binding region, a transmembrane domain and an intracellular region, the intracellular region consisting of a co-stimulatory domain, an intracellular signaling domain, and an additional signaling region,
wherein the additional signaling region consists of the intracellular region of the γc chain,
the intracellular signaling domain is the signaling domain of CD3 ζ,
the co-stimulatory domain is the co-stimulatory signaling domain of CD137 (4-1BB),
**characterized in that** the co-stimulatory domain, the intracellular signaling domain and the additional signaling region are arranged in order of distance from the cell membrane from nearest to farthest.

2. The chimeric antigen receptor according to claim 1, wherein the amino acid sequence of the intracellular region of the γc chain is as represented by SEQ ID NO: 16.

3. The chimeric antigen receptor according to claim 1, wherein the antigen-binding region is selected from the group consisting of sdAb, nanobody, antigen binding ligand, recombinant fibronectin domain, anticalin and DARPIN.

4. The chimeric antigen receptor according to claim 1, wherein the antigen-binding region is selected from the group consisting of monoclonal antibody, polyclonal antibody, recombinant antibody, human antibody, humanized antibody, murine antibody and chimeric antibody.

5. The chimeric antigen receptor according to any one of claims 1-2, wherein the antigen-binding region binds to a target selected from the group consisting of: TSHR, CD19, CD123, CD22, BAFF-R, CD30, CD171, CS-1, CLL-1, CD33, EGFRvIII, GD2, GD3, BCMA, GPRC5D, Tn Ag, PSMA, ROR1, FLT3, FAP, TAG72, CD38, CD44v6, CEA, EPCAM, B7H3, KIT, IL-13Ra2, mesothelin, IL-1 1Ra, PSCA, PRSS21, VEGFR2, LewisY, CD24, PDGFR-β, SSEA-4, CD20, Folate receptor α, ERBB2 (Her2/neu), MUC1, EGFR, NCAM, Claudin18.2, Prostase, PAP, ELF2M, Ephrin B2, IGF-I receptor, CAIX, LMP2, gp100, bcr-ab1, tyrosinase, EphA2, Fucosyl GM1, sLe, GM3, TGS5, HMWMAA, o-acetyl-GD2, Folate receptor β, TEM1/CD248, TEM7R, CLDN6, GPRC5D, CXORF61, CD97, CD 179a, ALK, polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, legumain, HPV E6, E7, MAGE A1, ETV6-AML, sperm protein 17, XAGE1, Tie 2, MAD-CT-1, MAD-CT-2, Fos associated antigen 1, p53, p53 mutant, prostate specific protein, survivin and telomerase, PCTA-1/Galectin 8, MelanA/MART1, Ras mutant, hTERT, sarcoma translocation breakpoint, ML-IAP, ERG (TMPRSS2 ETS fusion gene), NA17, PAX3, androgen receptor, Cyclin B1, MYCN, RhoC, TRP-2, CYP1B 1, BORIS, SART3, PAX5, OY-TES 1, LCK, AKAP-4, SSX2, RAGE-1, human telomerase reverse transcriptase, RU1, RU2, intestinal tract carboxylesterase, mut hsp70-2, CD79a, CD79b, CD72, LAIR1, FCAR, LILRA2, CD300LF, CLEC12A, BST2, EMR2, LY75, GPC3, FCRL5, IGLL1, PD1, PDL1, PDL2, TGF β, APRIL, NKG2D and any combination thereof.

6. The chimeric antigen receptor according to any one of claims 1-5, wherein the transmembrane domain is a transmembrane domain of a protein selected from the group consisting of: TCR α chain, TCR β chain, TCR γ chain, TCR δ chain, CD3 ζ subunit, CD3 ε subunit, CD3 γ subunit, CD3 δ subunit, CD45, CD4, CD5, CD8, CD9, CD16, CD22, CD33, CD28, CD37, CD64, CD80, CD86, CD134, CD137, and CD154.

7. A nucleic acid, **characterized by** containing a sequence encoding the chimeric antigen receptor according to any one of claims 1-6.

8. A vector, **characterized by** containing the nucleic acid according to claim 7.

9. An immune cell, **characterized by** containing the chimeric antigen receptor according to any one of claims 1-6, the nucleic acid according to claim 7, or the vector according to claim 8,
preferably, the vector is selected from the group consisting of linear nucleic acid molecule, plasmid, retrovirus, lentivirus, adenovirus, vaccinia virus, Rous Sarcoma Virus (RSV), polyoma virus and adeno-associated virus (AAV), bacteriophage, cosmid and artificial chromosome;
preferably, the immune cell is selected from the group consisting of a T cell, a macrophage, a dendritic cell, a monocyte, an NK cell and an NKT cell,
more preferably, the immune cell is a T cell selected from the group consisting of: CD4+/CD8+ double positive T cell, a CD4+ helper T cell, a CD8+ T cell, a tumor infiltrating cell, a memory T cell, a naive T cell, a γδ-T cell and an αβ-T cell.

10. The immune cell according to claim 9, wherein in the immune cell, at least one gene selected from the group consisting of CD52, GR, TCR α, TCR β, CD3 γ, CD3 δ, CD3 ε, CD247 ζ, HLA-I, HLA-II gene and immune checkpoint gene such as PD1 and CTLA-4 is inactivated, preferably by DNA breakage mediated by a meganuclease, a zincfinger nuclease, a TALE nuclease, or a Cas enzyme in a CRISPR system.

11. A pharmaceutical composition, **characterized by** containing the chimeric antigen receptor according to any one of claims 1-6, the nucleic acid according to claim 7, the vector according to claim 8, or the immune cell according to any one of claims 9-10, and one or more pharmaceutically acceptable excipients.

12. The pharmaceutical composition according to claim 11 for use in a method of treating a cancer selected from the group consisting of: blastoma, sarcoma, leukemia, basal cell carcinoma, biliary tract cancer, bladder cancer, bone cancer, brain and CNS cancer, breast cancer, peritoneal cancer, cervical cancer, choriocarcinoma, colon and rectal cancer, connective tissue cancer, cancer of digestive system, endometrial cancer, esophageal cancer, eye cancer, head and neck cancer, stomach cancer, glioblastoma (GBM), liver cancer, hepatoma, intraepithelial tumor, kidney cancer, larynx cancer, leukemia, liver tumor, lung cancer, lymphoma, melanoma, myeloma, neuroblastoma, oral cancer, ovarian cancer, pancreatic cancer, prostate cancer, retinoblastoma, rhabdomyosarcoma, rectal cancer, cancer of respiratory system, salivary gland cancer, skin cancer, squamous cell carcinoma, stomach cancer, testicular cancer, thyroid cancer, uterine or endometrial cancer, malignant tumor of urinary system, vulval cancer and other cancers and sarcomas, and B cell lymphoma, mantle cell lymphoma, AIDS-related lymphoma, and Waldenstrom macroglobulinemia, chronic lymphocytic leukemia (CLL), acute lymphocytic leukemia (ALL), B cell acute lymphocytic leukemia (B-ALL), T cell acute lymphocytic leukemia (T-ALL), B cell prolymphocytic leukemia, blast cell plasmacytoid dendritic cell tumor, Burkitt lymphoma, diffuse large B cell lymphoma, follicular lymphoma, chronic myelogenous leukemia (CML), malignant lymphoproliferative disorder, MALT lymphoma, hairy cell leukemia, marginal zone lymphoma, multiple myeloma, myelodysplasia, plasmablastic lymphoma, preleukemia, plasmacytoid dendritic cell tumor and post-transplant lymphoproliferative disorder (PTLD).

## Patentansprüche

1. Chimärer Antigenrezeptor, enthaltend eine Antigenbindungsregion, eine Transmembrandomäne und eine intrazelluläre Region, wobei die intrazelluläre Region aus einer kostimulatorischen Domäne, einer intrazellulären Signalisierungsdomäne und einer zusätzlichen Signalisierungsregion besteht,
wobei die zusätzliche Signalisierungsregion aus der intrazellulären Region der yc-Kette besteht,
die intrazelluläre Signalisierungsdomäne die Signalisierungsdomäne von CD3 ζ ist,
die kostimulatorische Domäne die kostimulatorische Signalisierungsdomäne von CD137 (4-1BB) ist,
**dadurch gekennzeichnet, dass** die kostimulatorische Domäne, die intrazelluläre Signalisierungsdomäne und die zusätzliche Signalisierungsregion in der Reihenfolge ihrer Entfernung von der Zellmembran vom nächsten zum entferntesten angeordnet sind.

2. Chimärer Antigenrezeptor nach Anspruch 1, wobei die Aminosäuresequenz der intrazellulären Region der yc-Kette wie durch SEQ ID NO: 16 dargestellt ist.

3. Chimärer Antigenrezeptor nach Anspruch 1, wobei die Antigenbindungsregion aus der Gruppe ausgewählt ist, die aus sdAb, Nanobody, Antigenbindungs-Ligand, rekombinanter Fibronektin-Domäne, Anticalin und DARPIN besteht.

4. Chimärer Antigenrezeptor nach Anspruch 1, wobei die Antigenbindungsregion aus der Gruppe ausgewählt ist, die aus monoklonalem Antikörper, polyklonalem Antikörper, rekombinantem Antikörper, humanem Antikörper, humanisiertem Antikörper, murinem Antikörper und chimärem Antikörper besteht.

5. Chimärer Antigenrezeptor nach einem der Ansprüche 1 bis 2, wobei die Antigenbindungsregion an ein Target bindet, das aus der Gruppe ausgewählt ist, die aus TSHR, CD19, CD123, CD22, BAFF-R, CD30, CD171, CS-1, CLL-1, CD33, EGFRvIII, GD2, GD3, BCMA, GPRC5D, Tn Ag, PSMA, ROR1, FLT3, FAP, TAG72, CD38, CD44v6, CEA, EPCAM, B7H3, KIT, IL-13Ra2, Mesothelin, IL-1 1Ra, PSCA, PRSS21, VEGFR2, LewisY, CD24, PDGFR-β, SSEA-4, CD20, Folatrezeptor α, ERBB2 (Her2/neu), MUC1, EGFR, NCAM, Claudin18.2, Prostase, PAP, ELF2M, Ephrin B2, IGF-I-Rezeptor, CAIX, LMP2, gp100, bcr-ab1, Tyrosinase, EphA2, Fucosyl GM1, sLe, GM3, TGS5, HMWMAA, o-acetyl-GD2, Folatrezeptor β, TEM1/CD248, TEM7R, CLDN6, GPRC5D, CXORF61, CD97, CD179a, ALK, Polysialinsäure, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, Legumain, HPV E6, E7, MAGE A1, ETV6-AML, Spermaprotein 17, XAGE1, Tie2, MAD-CT-1, MAD-CT-2, Fos-assoziiertem Antigen 1, p53, p53 mutierend, Prostataspezifisches Protein, Survivin und Telomerase, PCTA-1/Galectin 8, MelanA/MART1, Ras-Mutant, hTERT, Sarkom-Translokationsbruchpunkt, ML-IAP, ERG (TMPRSS2 ETS-Fusionsgen), NA17, PAX3, Androgenrezeptor, Cyclin B1, MYCN, RhoC, TRP-2, CYP1B1, BORIS, SART3, PAX5, OY-TES 1, LCK, AKAP-4, SSX2, RAGE-1, humane Telomerase-Reverse-Transkriptase, RU1, RU2, Darmtrakt Carboxylesterase, mutiertes hsp70-2, CD79a, CD79b, CD72, LAIR1, FCAR, LILRA2, CD300LF, CLEC12A, BST2, EMR2, LY75, GPC3, FCRL5, IGLLI1, PD1, PDL1, PDL2, TGF β, APRIL, NKG2D und jede Kombination davon, besteht.

6. Chimärer Antigenrezeptor nach einem der Ansprüche 1 bis 5, wobei die Transmembrandomäne eine Transmembrandomäne eines Proteins ist, das aus der Gruppe ausgewählt ist, die aus: TCR α-Kette, TCR β-Kette, TCR γ-Kette, TCR δ-Kette, CD3 ζ-Untereinheit, CD3 ε-Untereinheit, CD3 γ-Untereinheit, CD3 δ-Untereinheit, CD45, CD4, CD5, CD8, CD9, CD16, CD22, CD33, CD28, CD37, CD64, CD80, CD86, CD134, CD137 und CD154 besteht.

7. Nukleinsäure, **dadurch gekennzeichnet, dass** sie eine Sequenz enthält, die den chimären Antigenrezeptor nach einem der Ansprüche 1 bis 6 kodiert.

8. Vektor, **dadurch gekennzeichnet, dass** er die Nukleinsäure nach Anspruch 7 enthält.

9. Immunzelle, **dadurch gekennzeichnet, dass** sie den chimären Antigenrezeptor nach einem der Ansprüche 1 bis 6, die Nukleinsäure nach Anspruch 7 oder den Vektor nach Anspruch 8 enthält,
vorzugsweise wird der Vektor aus der Gruppe ausgewählt, die aus linearem Nukleinsäuremolekül, Plasmid, Retrovirus, Lentivirus, Adenovirus, Vaccinia-Virus, Rous-Sarkom-Virus (RSV), Polyomavirus und adeno-assoziiertem Virus (AAV), Bakteriophage, Kosmid und künstlichem Chromosom besteht;
vorzugsweise wird die Immunzelle aus der Gruppe ausgewählt, die aus einer T-Zelle, einem Makrophage, einer dendritischer Zelle, einem Monozyt, einer NK-Zelle und einer NKT-Zelle besteht,
besonders bevorzugt ist die Immunzelle eine T-Zelle, die aus der Gruppe ausgewählt ist, die aus: CD4+/CD8+ doppelt positiver T-Zelle, einer CD4+ T-Helfer-Zelle, einer CD8+ T-Zelle, einer tumorinfiltrierender Zelle, einer T-Gedächtniszelle, einer naiver T-Zelle, einer γδ-T-Zelle und einer αβ-T-Zelle besteht.

10. Immunzelle nach Anspruch 9, wobei mindestens ein Gen, das aus der Gruppe ausgewählt wird, die aus CD52, GR, TCR α, TCR β, CD3 γ, CD3 δ, CD3 ε, CD247 ζ, HLA-I, HLA-II-Gen und Immun-Checkpoint-Gen wie PD1 und CTLA-4 besteht, in der Immunzelle inaktiviert ist, vorzugsweise durch DNA-Bruch, der durch eine Meganuklease, eine Zinkfinger-Nuklease, eine TALE-Nuklease oder ein Cas-Enzym in einem CRISPR-System erreicht wird.

11. Pharmazeutische Zusammensetzung, **dadurch gekennzeichnet, dass** sie den chimären Antigenrezeptor nach einem der Ansprüche 1 bis 6, die Nukleinsäure nach Anspruch 7, den Vektor nach Anspruch 8 oder die Immunzelle nach einem der Ansprüche 9 oder 10 und ein oder mehrere pharmazeutisch akzeptable Exzipienten enthält.

12. Pharmazeutische Zusammensetzung nach Anspruch 11 zum Gebrauch in einem Verfahren zur Behandlung eines Krebses, das aus der Gruppe ausgewählt wird, die aus Blastom, Sarkom, Leukämie, Basalzellkarzinom, Gallengangskrebs, Blasenkrebs, Knochenkrebs, Gehirn- und ZNS-Krebs, Brustkrebs, Peritonealkrebs, Gebärmutterhalskrebs, Choriokarzinom, Dickdarm- und Rektumkrebs, Bindegewebskrebs, Krebs des Verdauungssystems, Endometriumkrebs, Speiseröhrenkrebs, Augenkrebs, Kopf- und Halskrebs, Magenkrebs, Glioblastom (GBM), Leberkrebs, Hepatom, intraepitheliale Tumoren, Nierenkrebs, Kehlkopfkrebs, Leukämie, Lebertumor, Lungenkrebs, Lymphom, Melanom, Myelom, Neuroblastom, Mundhölenkrebs, Eierstockkrebs, Bauchspeicheldrüsenkrebs, Prostatakrebs, Retinoblastom, Rhabdomyosarkom, Rektumkrebs, Krebs des Atmungssystems, Speicheldrüsenkrebs, Hautkrebs, Plattenepithelkarzinom, Magenkrebs, Hodenkrebs, Schilddrüsenkrebs, Gebärmutterkrebs oder Endometriumkrebs, malignen Tumoren des Harnsystems, Vulvakrebs und anderen Krebsarten und Sarkomen, sowie B-Zell-Lymphom, Mantelzell-Lymphom, AIDSassoziiertem Lymphom, Waldenström-Makroglobulinämie, chronischer lymphatischer Leukämie (CLL), akuter lymphatischer Leukämie (ALL), akuter lymphatischer B-Zell-Leukämie (B-ALL), akuter lymphatischer T-Zell-Leukämie (T-ALL), B-Zellprolymphozytischer Leukämie, blastischen plasmazytoiden dendritischen Zelltumoren, Burkitt-Lymphom, diffusem großzelligem B-Zell-Lymphom, follikulärem Lymphom, chronischer myeloischer Leukämie (CML), maligner lymphoproliferativer Erkrankung, MALT-Lymphom, Haarzell-Leukämie, Marginalzonen-Lymphom, multiplem Myelom, Myelodysplasie, plasmoblastischem Lymphom, Präleukämie, plasmazytoidem dendritischen Zelltumor und lymphoproliferativer Erkrankung nach Transplantation (PTLD) besteht.

## Revendications

1. Un récepteur antigénique chimérique, contenant une région de liaison à l'antigène, un domaine transmembranaire et une région intracellulaire, la région intracellulaire consistant en un domaine co-stimulateur, un domaine de signalisation intracellulaire, et une région de signalisation additionnelle,
où la région de signalisation additionnelle consiste en la région intracellulaire de la chaîne γc,
le domaine de signalisation intracellulaire étant le domaine de signalisation du CD3
le domaine co-stimulateur étant le domaine de signalisation co-stimulateur du CD137 (4-1BB),
**caractérisé en ce que** le domaine co-stimulateur, le domaine de signalisation intracellulaire et la région de signalisation additionnelle sont disposés par ordre de distance de la membrane cellulaire du plus proche au plus éloigné.

2. Le récepteur antigénique chimérique selon la revendication 1, où la séquence d'acides aminés de la région intracellulaire de la chaîne γc est représentée par SEQ ID NO: 16.

3. Le récepteur antigénique chimérique selon la revendication 1, où la région de liaison à l'antigène est choisie parmi le groupe constitué de sdAb, nanobody, ligand de liaison antigénique, domaine recombinant de fibronectine, anticaline et DARPIN.

4. Le récepteur antigénique chimérique selon la revendication 1, où la région de liaison à l'antigène est choisie parmi le groupe constitué d'anticorps monoclonal, anticorps polyclonal, anticorps recombinant, anticorps humain, anticorps humanisé, anticorps murin et anticorps chimérique.

5. Le récepteur antigénique chimérique selon l'une quelconque des revendications 1 ou 2, où la région de liaison à l'antigène se lie à une cible choisie parmi le groupe constitué de: TSHR, CD19, CD123, CD22, BAFF-R, CD30, CD171, CS-1, CLL-1, CD33, EGFRvIII, GD2, GD3, BCMA, GPRC5D, Ag Tn, PSMA, ROR1, FLT3, FAP, TAG72, CD38, CD44v6, CEA, EPCAM, B7H3, KIT, IL-13Ra2, mésothéline, IL-11Ra, PSCA, PRSS21, VEGFR2, LewisY, CD24, PDGFR-13, SSEA-4, CD20, récepteur de la folate α, ERBB2 (Her2/neu), MUC1, EGFR, NCAM, Claudin18.2, Prostase, PAP, ELF2M, Ephrin B2, récepteur IGF-I, CAIX, LMP2, gp100, bcr-ab1, tyrosinase, EphA2, GM1 fucosylé, sle, GM3, TGS5, HMWMAA, o-acétyl-GD2, récepteur de la folate β, TEM1/CD248, TEM7R, CLDN6, GPRC5D, CXORF61, CD97, CD179a, ALK, acide polysialique, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, légumaine, HPV E6, E7, MAGE A1, ETV6-AML, protéine du sperme 17, XAGE1, Tie 2, MAD-CT-1, MAD-CT-2, antigène associé au Fos 1, p53, mutant p53, protéine spécifique de la prostate, survivine et télomérase, PCTA-1/Galectin 8, MelanA/MART1, Ras mutant, hTERT, point de translocation du sarcome, ML-IAP, ERG (gène de fusion TMPRSS2 ETS), NA17, PAX3, récepteur des androgènes, Cycline B1, MYCN, RhoC, TRP-2, CYP1B1, BORIS, SART3, PAX5, OY-TES 1, LCK, AKAP-4, SSX2, RAGE-1, télomérase humaine inverse transcriptase, RU1, RU2, estérase intestinale, mut hsp70-2, CD79a, CD79b, CD72, LAIR1, FCAR, LILRA2, CD300LF, CLEC12A, BST2, EMR2, LY75, GPC3, FCRL5, IGLL1, PD1, PDL1, PDL2, TGF APRIL, NKG2D et toute combinaison de ceux-ci.

6. Le récepteur antigénique chimérique selon l'une quelconque des revendications 1 à 5, où le domaine transmembranaire est un domaine transmembranaire d'une protéine choisie parmi le groupe constitué de: chaîne α du TCR, chaîne β du TCR, chaîne γ du TCR, chaîne δ du TCR, sous-unité ζ du CD3, sous-unité ε du CD3, sous-unité γ du CD3, sous-unité δ du CD3, CD45, CD4, CD5, CD8, CD9, CD16, CD22, CD33, CD28, CD37, CD64, CD80, CD86, CD134, CD137, et CD154.

7. Un acide nucléique, **caractérisé en ce qu'**il contient une séquence codant pour le récepteur antigénique chimérique selon l'une quelconque des revendications 1 à 6.

8. Un vecteur, **caractérisé en ce qu'**il contient l'acide nucléique selon la revendication 7.

9. Une cellule immunitaire, **caractérisée en ce qu'**elle contient le récepteur antigénique chimérique selon l'une quelconque des revendications 1 à 6, l'acide nucléique selon la revendication 7, ou le vecteur selon la revendication 8,
de préférence, le vecteur est choisi parmi le groupe constitué de molécule d'acide nucléique linéaire, plasmide, rétrovirus, lentivirus, adénovirus, virus de la vaccine, virus du sarcome de Rous (RSV), polyomavirus et virus adéno-associé (AAV), bactériophage, cosmide et chromosome artificiel;
de préférence, la cellule immunitaire est choisie parmi le groupe constitué de cellule T, macrophage, cellule dendritique, monocyte, cellule NK et cellule NKT,
plus préférentiellement, la cellule immunitaire est une cellule T choisie parmi le groupe constitué de: cellule T double positive CD4+/CD8+, cellule T auxiliaire CD4+, cellule T CD8+, cellule infiltrante tumorale, cellule T mémoire, cellule T naïve, cellule γδ-T et cellule αβ-T.

10. La cellule immunitaire selon la revendication 9, où dans la cellule immunitaire, au moins un gène choisi parmi le groupe constitué de CD52, GR, TCR α, TCR β, CD3 γ, CD3 δ, CD3 ε, CD247 ζ, gène HLA-I, gène HLA-II et gène de point de contrôle immunitaire tel que PD1 et CTLA-4 est inactivé, de préférence par cassure d'ADN médiée par une méganucléase, une nucléase à doigt de zinc, une nucléase TALE, ou une enzyme Cas dans un système CRISPR.

11. Une composition pharmaceutique, **caractérisée en ce qu'**elle contient le récepteur antigénique chimérique selon l'une quelconque des revendications 1 à 6, l'acide nucléique selon la revendication 7, le vecteur selon la revendication 8, ou la cellule immunitaire selon l'une quelconque des revendications 9 ou 10, et un ou plusieurs excipients pharmaceutiquement acceptables.

12. La composition pharmaceutique selon la revendication 11 pour une utilisation dans un procédé de traitement d'un cancer choisi parmi le groupe constitué de: blastome, sarcome, leucémie, carcinome basocellulaire, cancer des voies biliaires, cancer de la vessie, cancer des os, cancer du cerveau et du système nerveux central (CNS), cancer du sein, cancer péritonéal, cancer du col de l'utérus, choriocarcinome, cancer du côlon et du rectum, cancer du tissu conjonctif, cancer du système digestif, cancer de l'endomètre, cancer de l'œsophage, cancer des yeux, cancer de la tête et du cou, cancer de l'estomac, glioblastome (GBM), cancer du foie, hépatome, tumeur intraépithéliale, cancer du rein, cancer du larynx, leucémie, tumeur hépatique, cancer du poumon, lymphome, mélanome, myélome, neuroblastome, cancer de l'ovaire, cancer du pancréas, cancer de la prostate, rétinoblastome, rhabdomyosarcome, cancer rectal, cancer du système respiratoire, cancer des glandes salivaires, cancer de la peau, carcinome épidermoïde, cancer des testicules, cancer de la thyroïde, cancer de l'utérus ou de l'endomètre, tumeur maligne du système urinaire, cancer de la vulve et autres cancers et sarcomes, ainsi que lymphome des cellules B, lymphome du manteau, lymphome associé au SIDA, macroglobulinémie de Waldenstrom, leucémie lymphoïde chronique (CLL), leucémie lymphoblastique aiguë (ALL), leucémie lymphoblastique aiguë des cellules B (B-ALL), leucémie lymphoblastique aiguë des cellules T (T-ALL), leucémie prolymphocytaire des cellules B, tumeur dendritique plasmacytoïde des cellules blastiques, lymphome de Burkitt, lymphome diffus à grandes cellules B, lymphome folliculaire, leucémie myéloïde chronique (CML), trouble lymphoprolifératif malin, lymphome MALT, leucémie à tricholeucocytes, lymphome de la zone marginale, myélome multiple, myélodysplasie, lymphome plasmoblastique, préleucémie, tumeur dendritique plasmacytoïde et trouble lymphoprolifératif post-transplantation (PTLD).
